Europäisches Patentamt

European Patent Office

Office européen des brevets

(11) Numéro de publication:

**0 094 285**
A1

## DEMANDE DE BREVET EUROPEEN

(21) Numéro de dépôt: 83400836.9

(22) Date de dépôt: 27.04.83

(51) Int. Cl.³: **A 61 K 7/48**, A 61 K 37/12, A 61 K 9/70

(30) Priorité: **07.05.82 FR 8208308**

(43) Date de publication de la demande: **16.11.83**
**Bulletin 83/46**

(84) Etats contractants désignés: **AT BE CH DE FR GB IT LI LU NL SE**

(71) Demandeur: **SANOFI, Société dite:, 40, Avenue George V, F-75008 Paris (FR)**

(72) Inventeur: **de Carcaradec, Nadège, 2 Place Rodin, F-75016 Paris (FR)**

(74) Mandataire: **Bressand, Georges et al, c/o CABINET LAVOIX 2 Place d'Estienne d'Orves, F-75441 Paris Cedex 09 (FR)**

(54) Préparation à base de collagène naissant lyophilisé et son utilisation dans le domaine cosmétique.

㉗ L'invention a pour objet une nouvelle forme d'utilisation topique de collagène et son utilisation dans le domaine cosmétologique pour combattre les phénomènes de vieillissement du tissu cutané.

## Préparation à base de collagène naissant lyophilisé et son utilisation dans le domaine cosmétique.-

La présente invention se rapporte à une nouvelle forme topique de collagène et à son utilisation dans le domaine cosmétologique.

Il est classiquement admis que le vieillissement du tissu cutané ainsi que la biogénèse de ses productions sont liés à l'activité des fonctions cellulaires spécifiques aux tissus. Pour tenter de maintenir ces fonctions en état d'activité physiologique, plusieurs solutions ont été proposées. C'est ainsi qu'ont été décrits des extraits tissulaires d'origine animale ou végétale, tels des extraits embryonnaires, des extraits végétaux, de l'élastine, des glycosaminoglycannes, etc, sans que les résultats obtenus soient valablement acceptables.

Le collagène est une protéine de soutien de la plupart des tissus conjonctifs et constitue environ 33 p.100 des protéines de l'être vivant. Au sein de ces tissus, le collagène joue un rôle considérable soit en physiologie, soit en pathologie. Grâce à sa structure, il permet la rigidité des fibres et il est aussi impliqué dans la cohérence des organes, la résistance à l'élongation et peut servir à l'adhésion des cellules. Il préside également à la différenciation des organes.

La macromolécule de collagène est constituée de trois chaînes peptidiques ayant chacune une masse de 100.000 daltons et se caractérise par une largeur de 15 Å et une longueur de 2990 Å. Les deux principales caractéristiques de sa composition en acides aminés sont d'une part la présence d'un résidu de glycocolle tous les trois résidus d'acides aminés et d'autre part la forte teneur en hydroxyproline qui permet l'exploration, chez l'homme, du métabolisme du collagène grâce au dosage urinaire de cet acide aminé.

Les trois chaînes peptidiques se présentent sous forme hélicoïdale et elles s'enroulent en hélice autour d'un axe commun qui est l'axe de la macromolécule.

Il existe naturellement différents types de collagène qui se différencient les uns des autres surtout par leur composition en acides aminés et parfois par l'arrangement des chaînes peptidiques les unes par rapport aux autres. C'est ainsi que le collagène se retrouve dans l'os, la dentine, les tendons, la peau, les tissus internes, le cartilage hyalin, certaines parties de l'oeil, les lames basales des vaisseaux, les membranes basales des glomérules rénaux, etc.

Les différents types de collagène peuvent être classés en plusieurs catégories, en fonction des caractéristiques précitées mais surtout en fonction du contrôle analytique qui s'exerce à trois niveaux :

- composition chimique,
- taille des macromolécules et liaisons réticulaires,
- structure.

C'est ainsi qu'ont été décrits des collagènes de type interstitiel -qui se retrouvent, par exemple, dans le cartilage hyalin des mammifères, dans la réticuline, dans les tendons, dans les os embryonnaires, etc.- et les collagènes des membranes basales -qui se retrouvent, par exemple, dans la capsule du cristallin de l'oeil, dans la peau, les os, les tissus embryonnaires, etc...-.

L'utilisation du collagène est dominée depuis de nombreuses années, par la fabrication du cuir et de la gélatine. Depuis quelques temps, le collagène intéresse les nutritionnistes, l'industrie pharmaceutique et l'industrie cosmétologique. Mais, cette utilisation pose un certain nombre de problèmes, étroitement liés aux critères requis pour obtenir un produit répondant de façon satisfaisante aux normes d'emploi dans le domaine considéré. Si l'on se référe, par exemple, au domaine thérapeutique, le collagène utilisé ne doit pas provoquer de réactions de sensibilisation et doit être parfaitement résorbable s'il est implanté dans l'organisme ; il ne doit être ni toxique ni cancérigène ; il peut alors être favorablement utilisé pour ses propriétés hémostatiques et cicatrisantes.

C'est ainsi qu'un collagène natif polymérisé a été mis au point et décrit (brevet français n°1.568.829, 1.596.789 et 1.596.790) et ses caractéristiques physico-chimiques ont été étudiés par des méthodes faisant appel à la diffraction des rayons X, à la chromatographie sur tamis moléculaire et à la calorimétrie différentielle programmée. Ce collagène provient du derme de la peau de veau, le derme ayant préalablement été débarrassé de ses constituants contaminants comme les Kératines, les mucopolysaccharides, les glycoprotéines et les graisses, par traitement chimique alcalin rapide. Après refendage mécanique -qui assure l'élimination de l'épiderme et la partie supérieure du derme, le produit restant subit différentes opérations (broyage, extraction par dispersion homogénéisation, précipitation, centrifugation, lavage à l'eau et à l'acétone, séchage, etc.) puis il est conditionné et stérilisé soit par l'oxyde d'éthylène, soit par irradiation gamma à doses de 2,5 - 2,8 Mrads, la chaleur étant proscrite dans tous les cas à cause de la dégradation du collagène. Ce collagène est fibreux et d'aspect cotonneux et il est pur à plus de 90 p.100. Par diffraction de rayons X, la courbe des intensités diffractées en fonction de l'angle de diffraction permet de mettre en évidence les pics $P_1$ et $P_2$ dont la présence assure de l'existence de collagène natif, c'est-à-dire non dénaturé.

Ce collagène a été utilisé en chirurgie cardiovasculaire, urologique, abdominale et neurologique, dans des types d'interventions précises et en vue d'obtenir une hémostase rapide, définitive et de bonne qualité et de manière telle que soit réduite la durée de l'intervention chirurgicale. De récents travaux d'une équipe de chirurgiens esthétiques de New-York ont décrit un nouveau procédé thérapeutique adjuvant pour effacer les cicatrices et les rides disgracieuses que la chirurgie ne peut enlever, à l'aide d'une préparation de collagène qui est injectée dans le derme, en solution aqueuse de lidocaïne : les résultats rapportés font état d'une efficacité variable mais la qualité et les caractéristiques du collagène utilisé ne sont pas indiqués.

La présente invention concerne donc une nouvelle forme d'utilisation topique du collagène natif précité, caractérisé en ce qu'il est présenté en plaques (ou masques-voile ou film) minces, blanchâtres et souples et uniquement constitués de collagène lyophilisé, sans aucun support. Après lyophilisation, et pour assurer leur parfaite aseptie et leur conservation, les plaques de collagène natif sont placées dans des poches stériles, ces plaques souples pouvant représenter des carrés de 20 à 25 cm de côté, ou de rectangles ayant de 5 à 10 cm de largeur et 20 à 25 cm de longueur par exemple.

Ces plaques, qui ont un toucher cotonneux, ont une faible épaisseur comprise entre 0,5 mm et 3 mm. Elles sont partiellement solubles dans l'eau distillée et complètement solubles en milieu acide.

Il va de soi qu'avant son utilisation dans le domaine cosmétique, cette préparation a subi les tests officiels d'analyse pour la détermination de l'indice d'irritation primaire sur le lapin, pour la détermination de l'irritation oculaire sur le lapin et pour l'appréciation de l'agressivité superficielle cutanée par applications itératives sur le lapin.

Ces tests, qui ont été satisfaisants et sont en faveur de la bonne tolérance de la préparation, ont été complétés par des essais de sensibilisation réalisés chez le rat, le lapin et le cobaye ; ils ont montré que l'administration par voie buccale et générale n'entraînait pas de réaction de sensibilisation.

La préparation de l'invention se caractérise également par des paramètres mécaniques qui permettent le maintien de l'intégrité totale de la plaque de collagène natif lyophilisée sans aucune rupture. Outre son pouvoir hémostatique, cette préparation se caractérise aussi par son action dans la croissance cellulaire ainsi que par ses propriétés de biocompatibilité et de biodégrabilité. Une autre caractéristique de l'invention concerne l'application de la préparation pour le traitement de beauté du cou, des yeux, du visage et du contour des lèvres, cette énumération étant donnée à titre d'exemples non limitatifs.

Après avoir démaquillé soigneusement les yeux, le visage et le cou, on effectue un léger gommage du visage et du cou à l'aide d'une crème spécialisée puis on rince avec des éponges imbibées d'eau tiède, cette opération étant suivie d'une pulvérisation d'un soluté tonique.

On humidifie le visage et le cou puis, après avoir retiré délicatement de son enveloppe stérile la préparation de l'invention, on plie ce masque-voile de manière à préparer les découpes adaptées au visage de la personne concernée, les découpes étant réalisées au niveau du nez et pour modeler le cou. On pose alors la préparation sur le visage et on hydrate progressivement à l'aide de 2 éponges imbibées d'un soluté tonique et on prend bien soin de commencer par le centre du cou et ensuite on remonte sur la zone médiane pour terminer par les zones externes. Cette hydratation doit être poursuivie jusqu'à ce que ce film de collagène natif adhère parfaitement à l'épiderme.

La préparation est maintenue in situ pendant 45 minutes environ et on prend soins de la réhydrater régulièrement toutes les 15 minutes à l'aide de pulvérisations ou d'éponges imbibées d'une solution tonique. On pose ensuite un mouchoir-papier plié dans le sens de la longueur au bas du film de collagène et on roule ce masque autour du papier de manière à le détacher de l'épiderme. On applique ensuite une crème.

A titre d'exemple non limitatif et pour illustrer la présente invention, il est décrit ci-dessous l'utilisation du film de collagène dans le traitement de beauté des yeux. Après avoir démaquillé soigneusement, on humidifie les paupières et les tempes avec une solution tonique. On ouvre l'enveloppe stérile contenant la préparation objet de l'invention et on retire ce masque de collagène que l'on place délicatement sur l'ensemble des yeux et des tempes. On hydrate comme précédemment à l'aide d'éponges imbibées de solution tonique jusqu'à ce que la plaque de collagène adhère parfaitement à l'épiderme. On laisse agir

pendant 45 minutes en ayant soin de réhydrater toutes les 10-15 minutes à l'aide d'éponges imbibées de solution tonique. On détache ensuite, comme décrit ci-dessus, le film de collagène de l'épiderme et on applique un gel de beauté.

Il y a lieu de préciser que tous les traitements de beauté décrits ci-dessus doivent être réalisés une fois par semaine pendant 5 semaines consécutives et qu'ils doivent être renouvelés au minimum 2 fois par an, ces indications étant données à titre non limitatif.

Les résultats obtenus à la suite de ces traitements cosmétologiques ont montré que la préparation de l'invention a été efficace pour combattre les phénomènes de vieillissement du tissu cutané qui a retrouvé sa souplesse et dont l'aspect a été favorablement modifié.

0094285

REVENDICATIONS

1. Nouvelle forme d'utilisation topique de collagène naissant lyophilisé partiellement soluble dans l'eau et complètement soluble en milieu acide, caractérisée en ce qu'elle est présentée sous forme d'un film.

2. Préparation selon la revendication 1, caractérisée en ce que le film a une épaisseur moyenne comprise entre 0,5 mm et 3 mm.

3. Préparation selon une des revendications 1 et 2, caractérisée en ce qu'elle est présentée sous enveloppe stérile.

4. Préparation selon une des revendications 1 à 3, caractérisée en ce qu'elle est présentée sous forme de film ayant une largeur comprise entre 5 et 25 cm et une longueur comprise entre 20 et 25 cm.

5. Préparation selon une des revendications 1 à 4, caractérisée en ce qu'elle est destinée à être appliquée sur l'épiderme humidifié du visage, du cou, des yeux et du contour des lèvres, à être hydratée progressivement et à être maintenue sur place pendant un laps de temps fonction du résultat à obtenir.

6. Préparation selon une des revendications 1 à 5, caractérisée en ce que le traitement correspondant doit être renouvelé 1 fois par semaine pendant 5 semaines consécutives, deux fois par an.

7. Préparation selon une des revendications 1 à 6, caractérisée en ce qu'elle est destinée à être utilisée dans le domaine cosmétologique et pour combattre les phénomènes de vieillissement du tissu cutané.

0094285

Office européen
des brevets

**RAPPORT DE RECHERCHE EUROPEENNE**

Numéro de la demande

EP  83 40 0836

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | FR-A-1 475 969 (F.M.C.) <br> * Résumé; page 1, colonne de droite, ligne 18 - page 2, colonne de gauche, ligne 18; page 3, colonne de gauche, lignes 17-39; page 4, colonne de gauche, lignes 16-23; exemple 1; page 4, colonne de droite, lignes 3-7 * <br><br> --- | 1,2,4 | A 61 K    7/48 <br> A 61 K   37/12 <br> A 61 K    9/70 |
| Y | FR-M-    6 652 (ROUVEIX) <br> * Résumé; page 2, colonne de droite, lignes 11-12 * <br><br> --- | 1,2 | |
| D,Y | FR-A-1 596 790 (CENTRE TECHNIQUE DU CUIR) <br> * Résumé * <br><br> --- | 1 | |
| Y | SEIFEN-ÖLE-FETTE-WACHSE, vol. 108, no. 7, avril 1982, pages 177-184, Augsburg, DE. <br> G. SCHUSTER et al.: "Proteinderivate in der Kosmetik - Welche und warum ? -" * Page 180, colonne de gauche, paragraphe 7 - colonne de droite, paragraphe 3; page 182, colonne de gauche, sous 2.1.1.; page 182, colonne de droite, sous 2.2.1 et 2.2.1.1. * <br><br> ---              -/- | 1,3,5-7 | DOMAINES TECHNIQUES RECHERCHES (Int. Cl. 3) <br><br> A 61 K    7/00 <br> A 61 K   37/00 <br> A 61 K    9/00 <br> A 61 L   15/00 <br> C 08 L   89/00 |

Le présent rapport de recherche a été établi pour toutes les revendications

| Lieu de la recherche <br> LA HAYE | Date d'achèvement de la recherche <br> 26-08-1983 | Examinateur <br> WILLEKENS G.E.J. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503. 03.82

Office européen
des brevets

**RAPPORT DE RECHERCHE EUROPEENNE**

Numéro de la demande

EP  83 40 0836

| | **DOCUMENTS CONSIDERES COMME PERTINENTS** | | Page  2 |
|---|---|---|---|
| Catégorie | Citation du document avec indication, en cas de besoin, des parties pertinentes | Revendication concernée | CLASSEMENT DE LA DEMANDE (Int. Cl. ³) |
| Y | DE-A-2 462 221  (TROMMSDORFF)<br><br>* Revendication; page 4, ligne 5 - page 7, ligne 3 * | 1,3,5-7 | |
| Y | <br><br>* Revendications; page 9, lignes 1-10 *<br><br>--- | 1,3,5-7 | |
| Y | FR-A-2 307 521  (C. FREUDENBERG)<br>* Revendications; page 1, lignes 15-32 *<br><br>--- | 1,5,7 | |
| Y | FR-A-2 307 971  (C. FREUDENBERG)<br>* Revendications *<br><br>--- | 1 | |
| Y | FR-A-2 120 010  (C.L.R.)<br>* Revendications; page 2, lignes 10-22; page 3, lignes 25-33 *<br><br>--- | 1 | DOMAINES TECHNIQUES RECHERCHES (Int. Cl. ³) |
| A | FR-A-2 120 295  (BALDON)<br>* Revendications; page 1, lignes 32-33; page 3, lignes 20-29 *<br><br>--- | 2,4 | |
| A | DE-A-1 467 886  (FITZKE)<br>* Revendications;  figures; page 2,  dernier  alinéa  -  page  3, dernier alinéa *<br><br>---                     -/- | 3-5 | |
| | Le présent rapport de recherche a été établi pour toutes les revendications | | |

| Lieu de la recherche<br>LA HAYE | Date d'achèvement de la recherche<br>26-08-1983 | Examinateur<br>WILLEKENS G.E.J. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82

Office européen
des brevets

**RAPPORT DE RECHERCHE EUROPEENNE**

Numéro de la demande

EP 83 40 0836

| | **DOCUMENTS CONSIDERES COMME PERTINENTS** | | Page 3 |
|---|---|---|---|
| Catégorie | Citation du document avec indication. en cas de besoin, des parties pertinentes | Revendication concernée | CLASSEMENT DE LA DEMANDE (Int. Cl. ³) |
| P,Y | FR-A-2 503 561 (SETON CY.)<br>* Revendications 1,2; page 2, lignes 14-20; exemple 1 *<br><br>-----  | 1,5 | |
| | | | **DOMAINES TECHNIQUES RECHERCHES (Int. Cl. ³)** |

Le présent rapport de recherche a été établi pour toutes les revendications

| Lieu de la recherche<br>LA HAYE | Date d'achèvement de la recherche<br>26-08-1983 | Examinateur<br>WILLEKENS G.E.J. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

&: membre de la même famille, document correspondant

OEB Form 1503. 03.82